# EUROPEAN PATENT APPLICATION

(11) **EP 3 791 829 A1**
(43) Date of publication of application: **17.03.2021**
(21) Application number: 19894168.4
(22) Date of filing: 20.11.2019
(51) Int. Cl.: A61F 2/42

(54) **TOE JOINT PROSTHESIS AND MANUFACTURING METHOD THEREFOR**

(30) Priority: 04.12.2018 CN 201811472414
(71) Applicant: Beijing Chunlizhengda Medical Instruments Co., Ltd, Beijing 101112 (CN)
(72) Inventor: SHI, Chunbao, Beijing 101112 (CN); XU, Kuixue, Beijing 101112 (CN); LU, Xiaoqiang, Beijing 101112 (CN); DONG, Zeyu, Beijing 101112 (CN); SHI, Wenchao, Beijing 101112 (CN); XIE, Fengbao, Beijing 101112 (CN); WANG, Zhenguo, Beijing 101112 (CN)
(74) Representative: Huang, Liwei
(86) International application number: PCT/CN2019/119617
(87) International publication number: WO 2020/114252

(57) **Abstract**

A toe joint prosthesis and a fabrication method thereof are provided. The toe joint prosthesis includes a first bone nail prosthesis and a second bone nail prosthesis hinged with the first bone nail prosthesis, the first bone nail prosthesis includes a first bone nail configured to be provided in a bone marrow cavity and a second bone nail connected with the first bone nail and located outside the bone marrow cavity, and the second bone nail is hinged with the second bone nail prosthesis. In particular, a three-dimensional porous structure layer is formed on an outer surface of the first bone nail and/or the second bone nail and/or the second bone nail prosthesis. An outer surface of the toe joint prosthesis are endowed with better bone ingrowth ability and bone crawling ability, thereby making the fixation of the toe joint prosthesis more stable.

## Description

### CROSS-REFERENCE TO RELATED APPLICATION

This application claims the priority benefit of China patent application No. 201811472414.9 titled "Toe joint prosthesis" filed on December 4, 2018 by Beijing Chunlizhengda Medical Instruments Co., Ltd. The entirety of the above-mentioned patent application is hereby incorporated by reference herein.

### TECHNICAL FIELD

The present application relates to the field of medical prosthesis, and, in particular, to a toe joint prosthesis and a fabrication method thereof.

### DESCRIPTION OF RELATED ART

A toe joint prosthesis is a kind of surgical implant used to replace the wounded toe joint of a human patient during toe joint replacement operation. When the human toe joint needs to be repaired due to tumor, comminuted fracture or the like, it is easy to cause serious bone defect of the toe joint. The serious bone defect not only degrades the bearing capacity of the osteotomy position of the toe joint, but also easily leads to an attachment point defect or joint capsule damage of the muscle at the toe joint. At this time, it is necessary to select a suitable prosthesis to replace the wounded toe joint.

The existing toe joint prosthesis is mainly composed of a first bone nail prosthesis and a second bone nail prosthesis. The first bone nail prosthesis and the second bone nail prosthesis are hinged to realize moving function of the toe joint prosthesis. However, the first bone nail prosthesis and the second bone nail prosthesis of the existing toe joint prosthesis, on one hand, can not be effectively fixed with the osteotomy segments of the wounded area; and on the other hand, the attachment point and joint capsule of the muscle near the toe joint prosthesis can not more effectively reconstructed, which can not ensure the stability of implanted toe joint prosthesis, not being conducive to the postoperative rehabilitation of the patient, or even causing secondary injury to the patient.

In view of the deficiency present in the prior art, it is urgent for those skilled in the art to seek a more stable toe joint prosthesis after implantation, so as to address the deficiency present in the prior art.

### SUMMARY

In order to make an implanted toe joint prosthesis more stable, the present application provides a toe joint prosthesis and a fabrication method thereof.

The toe joint prosthesis according to an embodiment of the present application includes: a first bone nail prosthesis and a second bone nail prosthesis hinged with the first bone nail prosthesis. The first bone nail prosthesis includes a first bone nail configured to be provided in a bone marrow cavity and a second bone nail connected with the first bone nail and located outside the bone marrow cavity. The second bone nail is hinged with the second bone nail prosthesis. In particular, a three-dimensional porous structure layer is formed on an outer surface of the first bone nail and/or the second bone nail and/or the second bone nail prosthesis.

Further, the toe joint prosthesis is a foot toe joint prosthesis, in which the first bone nail prosthesis is configured to be connected with a metatarsal bone, the second bone nail prosthesis is configured to be connected with a phalanx, the first bone nail is located in the bone marrow cavity of the metatarsal bone, and the second bone nail is located outside the bone marrow cavity of the metatarsal bone and abuts against an end face of the metatarsal bone.

Further, the toe joint prosthesis is a metacarpophalangeal joint prosthesis, in which the first bone nail prosthesis is configured to be connected a the metacarpal bone, the second bone nail prosthesis is configured to be connected with a phalangeal bone, the first bone nail is located in the bone marrow cavity of the metacarpal bone, and the second bone nail is located outside the bone marrow cavity of the metacarpal bone and abuts against an end face of the metacarpal bone.

Further, the three-dimensional porous structure layer includes a plurality of struts and a plurality of pores formed by staggered connection of the plurality of struts. The pores are communicated with each other, and have different average diameters. In particular, the average diameter of the pores ranges from 100 µm to 400 µm, and the porosity of the three-dimensional porous structure layer ranges from 50% to 80%.

Further, the average diameter of the pores in the three-dimensional porous structure layer formed on the outer surface of the first bone nail is larger than that of the pores in the three-dimensional porous structure layer formed on an outer surface of the second bone nail.

Further, the first bone nail prosthesis includes a hinge joint fixedly connected with the second bone nail. One end of the hinge joint facing the second bone nail prosthesis is configured as a spherical surface. The second bone nail prosthesis includes a spherical concave portion, and an end face of the spherical concave portion facing the hinge joint is a concave spherical surface configured to be matched with the spherical surface.

Further, the second bone nail prosthesis includes a needle-shaped portion connected with the spherical concave portion. The spherical concave portion further includes an outwardly protruded arc surface connected between the concave spherical surface and the peripheral wall of the needle-shaped portion, and the three-dimensional porous structure layer is formed on the arc surface.

Further, the arc surface of the spherical concave portion includes a first surface configured for extending into the bone marrow cavity and a second surface located outside the bone marrow cavity. In particular, the average diameter of the pores in the three-dimensional porous structure layer formed on the first surface is greater than the average diameter of the pores in the three-dimensional porous structure layer formed on the second surface.

Further, the average diameter of the pores in the three-dimensional porous structure layer formed on the first surface ranges from 100 µm to 200 µm, and the average diameter of the pores in the three-dimensional porous structure layer formed on the second surface ranges from 200 µm to 400 µm.

Further, a first suture hole extending through the second bone nail is formed on a side wall of the second bone nail, and a second suture hole extending through the hinge joint is formed on an end of the hinge joint near the second bone nail.

Further, an outer surface of the needle-shaped portion is a rough surface.

Further, the first bone nail, the second bone nail and the three-dimensional porous structure layer are integrally formed, and/or, the second bone nail prosthesis and the three-dimensional porous structure layer are integrally formed.

A fabrication method of a toe joint prosthesis according to the present application includes the following steps:
Step 1, forming a three-dimensional porous structure layer on an outer surface of a first bone nail and/or second bone nail and/or a second bone nail prosthesis;
Step 2, disposing the first bone nail in a bone marrow cavity, disposing the second bone nail outside the bone marrow cavity, and connecting the first bone nail with the second bone nail to form a first bone nail prosthesis; and
Step 3, hinging the first bone nail prosthesis with the second bone nail prosthesis to form a toe joint prosthesis.

Further, the toe joint prosthesis is a foot toe joint prosthesis. In particular, the first bone nail prosthesis is configured to be connected with a metatarsal bone, the second bone nail prosthesis is configured to be connected with a phalanx, the first bone nail is located in the bone marrow cavity of the metatarsal bone, and the second bone nail is located outside the bone marrow cavity of the metatarsal bone and abuts against an end face of the metatarsal bone.

Further, the toe joint prosthesis is a metacarpophalangeal joint prosthesis. In particular, the first bone nail prosthesis is configured to be connected with a metacarpal bone, the second bone nail prosthesis is configured to be connected with a phalangeal bone, the first bone nail is located in the bone marrow cavity of the metacarpal bone, and the second bone nail is located outside the bone marrow cavity of the metacarpal bone and abuts against an end face of the metacarpal bone.

Further, in Step 1, forming a three-dimensional porous structure layer on an outer surface of a first bone nail and/or a second bone nail and/or a second bone nail prosthesis includes the steps of:
scanning the first bone nail and/or the second bone nail and/or the second bone nail prosthesis by using a micro CT, and remodeling the scanned data by using MIMICS to obtain a three-dimensional schematic model;
dividing the three-dimensional schematic model into different regions which are adapted for different growth requirements of the same tissue, and further adjusting the diameter of the pores in individual regions so that different regions have different porosity;
generating a solid model of the first bone nail and/or the second bone nail and/or the second bone nail prosthesis by using a 3D printing equipment, in which the focus offset parameter of the 3D printing device is adjusted so that a plurality of bumps are formed on the surface of struts in the generated solid model, and the value of the focus offset parameter ranges from 5.8 mA to 6.2 mA.

Further, the average diameter of the pores in the three-dimensional porous structure layer formed on an outer surface of the first bone nail is greater than that of the pores in the three-dimensional porous structure layer formed on an outer surface of the second bone nail.

Further, the first bone nail prosthesis includes a hinge joint fixedly connected with the second bone nail, one end of the hinge joint facing the second bone nail prosthesis is configured as a spherical surface, the second bone nail prosthesis includes a spherical concave portion, and an end face of the spherical concave portion facing the hinge joint is a concave spherical surface matched with the spherical surface.

Further, the second bone nail prosthesis includes a needle-shaped portion connected with the spherical concave portion, and the spherical concave portion further includes an outwardly protruded arc surface connected between the concave spherical surface and the peripheral wall of the needle-shaped portion, and the three-dimensional porous structure layer is formed on the arc surface.

Further, the arc surface of the spherical concave portion includes a first surface configured for extending into the bone marrow cavity and a second surface located outside the bone marrow cavity, in which the average diameter of the pores in the three-dimensional porous structure layer formed on the first surface is greater than the average diameter of the pores in the three-dimensional porous structure layer formed on the second surface.

Further, the average diameter of the pores in the three-dimensional porous structure layer formed on the first surface ranges from 100 µm to 200 µm, and the average diameter of the pores in the three-dimensional porous structure layer formed on the second surface ranges from 200 µm to 400 µm.

Further, a first suture hole extending through the second bone nail is formed on a side wall of the second bone nail, and a second suture hole extending through the hinge joint is formed on one end of the hinge joint near the second bone nail.

Further, the outer surface of the needle-shaped portion is a rough surface.

Further, the first bone nail, the second bone nail and the three-dimensional porous structure layer are integrally formed, and/or, the second bone nail prosthesis and the three-dimensional porous structure layer are integrally formed.

Compared with the prior art, the toe joint prosthesis of the present application has the following advantages:
1) Forming a three-dimensional porous structure layer on the outer surface of the first bone nail prosthesis and/or the second bone nail prosthesis endows the outer surface of the first bone nail prosthesis and/or the second bone nail prosthesis with better bone growth ability and bone crawling ability, rendering the fixation of the toe joint prosthesis more stable.
2) The three-dimensional porous structure layer can also effectively reduce the weight of the toe joint prosthesis, and facilitate the reconstruction of an attachment point of soft tissues and muscles, which can effectively reduce the postoperative recovery time of a patient, and greatly improve the postoperative recovery effect of the patient.
3) Customized production of the first bone nail prosthesis and the second bone nail prosthesis of the toe joint prosthesis according to the present application can not only adapt them to the inner diameter and bending curvature of the bone marrow cavity of the patient, but also facilitate restoring the length of the toe joint, so as to ensure that the length of the wounded toe joint of the patient is consistent with that of the healthy toe joint.
4) Providing the first suture hole and the second suture hole is also helpful for repairing or reconstructing damaged joint capsules, so as to facilitate the postoperative recovery of the patient.

### BRIEF DESCRIPTION OF DRAWINGS

In order to more clearly explain technical solutions provided the Detailed Description of the present application or in the prior art, drawings needed for describing Detailed Description of the present application or the prior art will be briefly described. In all drawings, similar elements or parts are generally identified by similar reference signs. In the drawings, the elements or parts are not necessarily drawn to actual scale.
FIG. 1 is a structural diagram of the toe joint prosthesis according to the present application;
FIG. 2 is a schematic diagram of the connection of the toe joint prosthesis with a metatarsal bone and a phalanx shown in FIG. 1;
FIG. 3 is a structural schematic diagram of the first bone nail prosthesis shown in FIG. 1;
FIG. 4 is a structural schematic diagram of the hinge joint shown in FIG. 3; and
FIG. 5 is a structural schematic diagram of the second bone nail prosthesis shown in FIG. 1.

### DETAILED DESCRIPTION

Embodiments of the technical solutions of the present application will be described in detail in combination with Drawings. The following embodiments are only provided for explaining the technical solutions of the present application more clearly, so it is only used as an example and not intended to limit the protection scope of the present application in any way.

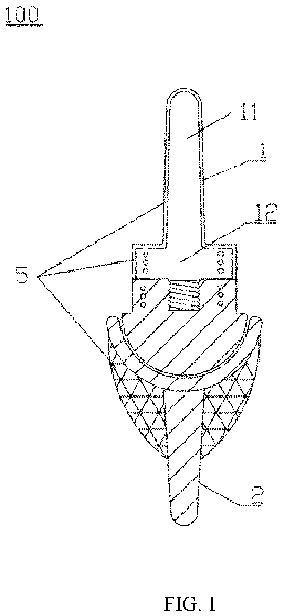

Fig. 1 shows the structure of a toe joint prosthesis 100 according to the present application. As shown in Fig. 1, the toe joint prosthesis 100 includes: a first bone nail prosthesis 1 and a second bone nail prosthesis 2 hinged with the first bone nail prosthesis 1. The first bone nail prosthesis 1 includes a first bone nail 11 provided in a bone marrow cavity and a second bone nail 12 which is connected with the first bone nail 11, located outside the bone marrow cavity, and hinged with the second bone nail prosthesis 2. In particular, a three-dimensional porous structure layer 5 is formed on an outer surface of the first bone nail 11 and/or the second bone nail 12 and/or the second bone nail prosthesis 2. It should be noted that, the toe joint prosthesis 100 of the present application can be a foot toe joint prosthesis or a metacarpophalangeal joint prosthesis. The structure and use method of the toe joint prosthesis 100 of the present application will be specifically described below by taking a foot toe joint prosthesis as an example.

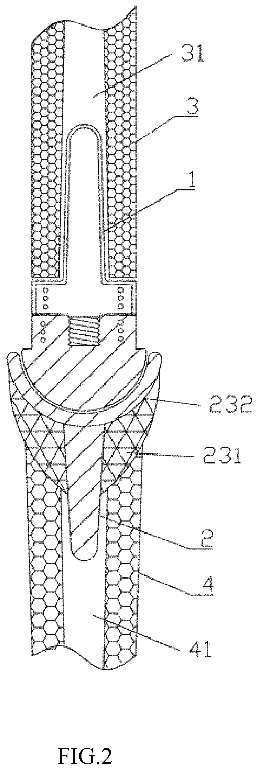

As shown in FIG. 1 and FIG 2, the toe joint prosthesis 100 is a foot toe joint prosthesis, the first bone nail prosthesis 1 is configured to be connected with a metatarsal bone 3, the second bone nail prosthesis 2 is configured to be connected with a phalanx 4, the first bone nail 11 is located in the bone marrow cavity of the metatarsal bone 3, and the second bone nail 12 is located outside the bone marrow cavity of the metatarsal bone 3 and abuts against an end face of the phalanx 4.

It should be noted that, when the toe joint prosthesis 100 is a metacarpophalangeal joint prosthesis, the first bone nail prosthesis 1 is configured to be connected with a metacarpal bone, the second bone nail prosthesis 2 is configured to be connected with a phalangeal bone, the first bone nail 11 is located inside the bone marrow cavity of the metacarpal bone, and the second bone nail 12 is located outside the bone marrow cavity of the metacarpal bone and abuts against an end face of the metacarpal bone.

As shown in FIG. 2, when the foot toe joint prosthesis of the present application is applied for replacing a foot toe joint, osteotomy is required for the metatarsal bone 3 and the phalanx 4 of a patient, after which there are a lot of bone defects in the metatarsal bone 3 and the phalanx 4. In some embodiments, the first bone nail prosthesis 1 is configured to include a first bone nail 11 and a second bone nail 12, so that the first bone nail 11 can be extended into the bone marrow cavity 31 of a metatarsal bone 3 and quickly fused with bone tissues in the bone marrow cavity 31. Meanwhile, the second bone nail 12 outside the metatarsal bone 3 can effectively make up for bone defects of metatarsal bone 3, that is, the length of the second bone nail 12 can make up for the length of the bone defects of the metatarsal bone 3. At the same time, the second bone nail 12 abuts against an end face of the metatarsal bone 3, which can effectively ensure the stability of the fixation between the first bone nail prosthesis 1 and the metatarsal bone 3. Connecting the second bone nail prosthesis 2 at one end with the phalanx 4 and hinging the second bone nail prosthesis 2 with the first bone nail prosthesis 1 at the other end achieves the connection between the second bone nail prosthesis 2 and the metatarsal bone 3 as well as the sliding movement of the phalanx 4 relative to metatarsal bone 3, so as to realize the moving function of the toe joint 100.

Forming a three-dimensional porous structure layer 5 on the outer surface of the first bone nail 11 and/or the second bone nail 12 and/or the second bone nail prosthesis 2 makes the outer surface of the first bone nail prosthesis 1 and/or the second bone nail prosthesis 2 a porous structure. In this way, the first bone nail 11 has a larger contact area with the inner wall of the bone marrow cavity 31, that is, the outer surface of the first bone nail 11 has better bone ingrowth ability, so that the bone tissue in the bone marrow cavity 31 can grow into the porous structure quickly, facilitating a stable fixation of the toe joint prosthesis 100 in the metatarsal bone 3. The porous structure formed on the outer surface of the second bone nail 12 endows the outer surface of the second bone nail 12 with better bone crawling ability, so that bone tissues in the bone marrow cavity 31 can quickly grow into the porous structure along the outer surface of the second bone nail 12, rendering the fixation of first bone nail prosthesis 1 with the metatarsal bone 3 more stable. Also, the porous structure formed on the outer surface of the second nail prosthesis 2 endows the second bone nail prosthesis 2 with a larger contact area with the phalanx 4, so that bone tissues in bone marrow cavity 41 can grow into the porous structure quickly, which endows the outer surface of the second bone nail prosthesis 2 with better bone ingrowth and crawling abilities, and renders the fixation of the second bone nail prosthesis 2 with the phalanx 4 more stable.

In addition, the providing of the three-dimensional porous structure layer 5 can effectively reduce the weight of the toe joint prosthesis 100, and facilitates the reconstruction of the attachment points of soft tissues and muscles, which can effectively reduce the postoperative recovery time of a patient, and greatly improve the recovery effect of the patient.

In addition, customized production of the first bone nail prosthesis 1 and the second bone nail prosthesis 2 makes the first bone nail prosthesis 1 and second bone nail prosthesis 2 more adaptive to the inner diameter and bending curvature of the bone marrow cavities of the metatarsal bone 3 and the phalanx 4 of the patient, thus further strengthening the stability of the matching between the first bone nail prosthesis 1 and the metatarsal bone 3, and the second bone nail prosthesis 2 and the phalanx 4. Further, customized first bone nail prostheses 1 and second bone nail prostheses 2 is helpful for restoring the length of a foot toe joint, thus ensuring that the length of the wounded foot toe joint is consistent with that of a healthy foot toe joint.

According to the present application, the three-dimensional porous structure layer 5 can include a plurality of struts and a plurality of pores formed by staggered connection of the plurality of struts, in which the pores are communicated with each other and have different average diameters. In particular, the average diameter of the pores ranges from 100 µm to 400 µm, and the porosity of the three-dimensional porous layer 5 ranges from 50% to 80%. By communicating the pores formed by staggered connection of struts with each other, providing different average diameter of the pores, and setting the above average diameter and porosity range of the three-dimensional porous structure layer 5, the structure of the three-dimensional porous structure layer 5 more resembles the bone trabecular structure of a human body, and the porous structure layer 5 having high porosity and connectivity can well induce the bone to grow into the pores of the three-dimensional porous structure layer 5 quickly and naturally, thus improving the adhesion, proliferation and differentiation of osteoblasts, effectively promoting the ingrowth and crawling of bone tissues in metatarsal bone 3 and the phalanx 4, which is conducive to the rapid fusion and fixation of the toe joint prosthesis 100 with human bone tissues after an operation. Therefore, it can effectively improve the postoperative recovery effect of the patient.

Preferably, the density of the pores in the direction from the outside to the inside of the three-dimensional porous structure layer 5 is gradually increased, so that the bone tissue can grow more smoothly from the outside of the three-dimensional porous structure layer 5 into the interior of the three-dimensional porous structure layer 5, so as to improve the fusion and fixation effect of the three-dimensional porous structure layer 5 with human bone tissues.

Further preferably, a plurality of convex portions (not shown) can be formed on the peripheral wall of the struts, which are configured to increase the roughness of the peripheral wall of the struts, so as to increase the friction force of the peripheral wall. In this way, the fixation of bone tissues with the struts is more firm and stable during bone growth. Preferably, the convex portion can be a circular convex point or a conical convex point. It should be noted that the shape of the convex portions is not limited to the above-mentioned shapes, as long as the convex portions can effectively increase the outer surface area of the struts to achieve the purpose of increasing the friction force of its peripheral wall, which will not be repeated here. Further, the width of the maximum profile shape of the cross section of the convex portions ranges 5-50 µm and the maximum height thereof ranges 10-50 µm.

Further preferably, the convex portions can be evenly distributed along the outer surface of the struts, or can be distributed in a discrete manner. Even further preferably, the number of convex portions on the struts in an area of the three-dimensional porous structure 5 with larger porosity may be greater than the number of the convex portions on the struts in an area of the three-dimensional porous structure 5 with smaller porosity, so as to make the bone ingrow more firmly. Further preferably, the density of the convex portions on the struts at an outer edge of the three-dimensional porous structure 5 is greater than that of the convex portions on the struts at the interior of the three-dimensional porous structure layer 5.

In a preferred embodiment, a three-dimensional porous structure layer 5 is formed on the outer surfaces of both the first bone nail 11 and the second bone nail 12. Preferably, the three-dimensional porous structure layers 5 formed on the outer surface of the first bone nail 11 and the second bone nail 12 can have the same thickness. Preferably, the thickness of the three-dimensional porous structure layer 5 ranges from 1 mm to 2 mm, more preferably 1.5 mm. Through this arrangement, on the one hand, the first bone nail prosthesis 1 is provided with an outer surface which is more conducive to bone ingrowth and bone crawling, and, on the other hand, the first bone nail prosthesis 1 is provided with better stress load bearing, conducting and dispersing capacity, so that the toe joint prosthesis 100 has more stable use effect.

Of course, the three-dimensional porous structure layers 5 formed on the outer surfaces of the first bone nail 11 and the second bone nail 12 can have different thicknesses, which can be specifically designed according to different patients to which the first bone nail prosthesis 1 is applied. For example, when a patient has poor bone ingrowth ability, the thickness of the three-dimensional porous structure layer 5 on the outer surface of the first bone nail 11 can be configured to be larger than the thickness of the three-dimensional porous structure layer 5 on the outer surface of the second bone nail 12, so as to ensure the strength of the first bone nail prosthesis 1. When a patient has poor bone crawling ability, the thickness of the three-dimensional porous structure layer 5 on the outer surface of the second bone nail 12 can be configured to be thicker than that of the three-dimensional porous structure layer 5 on the outer surface of the first bone nail 11, in order to ensure the stability of the fixation of the first bone nail prosthesis 1.

Preferably, the average diameter of the pores in the three-dimensional structure layer 5 formed on the outer surface of the first bone nail 11 may be larger than the average diameter of the pores in the three-dimensional structure layer 5 formed on the outer surface of the second bone nail 12. Further, the average diameter of the pores in the three-dimensional porous structure layer 5 formed on the outer surface of the first bone nail 11 preferably ranges from 200 µm to 400 µm for which the porosity of this region can be controlled between 50% and 60%, further preferably from 300 µm to 400 µm for which the porosity can be controlled between 50% and 55%. The average diameter of the pores preferably ranges from 100 µm to 200 µm for which the porosity can be controlled between 70% and 80%, further preferably from 100 µm to 150 µm for which the porosity can be controlled between 75% and 80%.

Through the above arrangement, when the diameter of the three-dimensional porous structure layer 5 formed on the outer surface of the first bone nail 11 ranges from 200 µm to 400 µm, bone ingrowth can be further promoted, so as to achieve a more stable fixation effect in the initial stage of replacement of the toe joint prosthesis 100. When the diameter of the three-dimensional porous structure layer 5 formed on the outer surface of the second bone nail 12 ranges from 100 µm to 200 µm, the growth speed by bone crawling is the fastest, so as to accelerate the growth of bone tissues in the middle and late stages of the replacement of the toe joint prosthesis 100, and in turn promote a fast recovery of the patient.

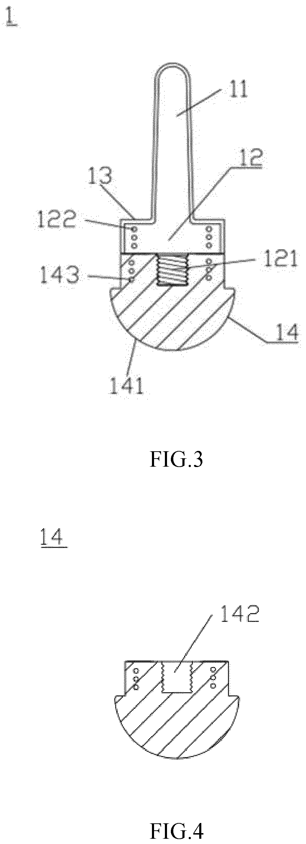

As shown in FIG. 3, a platform 13 configured for abutting against an end face of the metatarsal bone 3 can be formed at the joint of the second bone nail 12 and the first bone nail 11. The platform 13 is used to carry the force from the metatarsal bone 3, which realizes a better mechanical bearing function of the second bone nail 12 in the form of a platform, so as to improve the overall mechanical bearing capacity of the first bone nail prosthesis 1. Preferably, the second bone nail 12 can be configured to be a cylindrical shape with a circular cross section, so that the toe joint prosthesis 100 is more stable in use and has a more stable supporting effect for the metatarsal bone 3.

In another preferred embodiment, a three-dimensional porous structure layer 5 is formed on the outer surfaces of the first bone nail 11, the second bone nail 12 and the second bone nail prosthesis 2. The thickness of the three-dimensional porous structure layer 5 formed on the outer surface of the second bone nail prosthesis 2 can be the same as or different from the thickness of the three-dimensional porous structure layer 5 on the outer surface of the first bone nail 11 and the second bone nail 12, which can be designed according to different patients to which the second bone nail prosthesis 2 is applied. The structure of this part will be described in detail below.

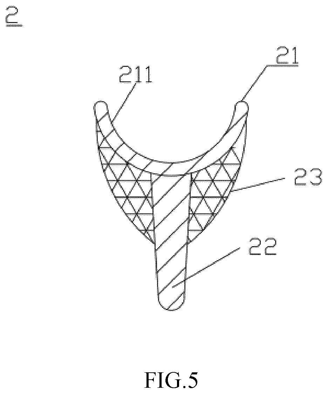

According to the present application, as shown in FIG. 3, the first bone nail prosthesis 1 can further include a hinge joint 14 fixedly connected with the second bone nail 12, in which one end of the hinge joint 14 facing the second bone nail prosthesis 2 is configured as a spherical surface 141. As shown in FIG. 5, the second bone nail prosthesis 2 includes a spherical concave portion 21, and an end face of the spherical concave portion 21 facing the hinge joint 14 is provided with a concave spherical surface 211 matched with the spherical surface 141. Through the matching of spherical surface 141 of hinge joint 14 and concave spherical surface 211 of second bone nail prosthesis 2, a spherical hinge connection mode is formed between hinge joint 14 and the second bone nail prosthesis 2. Such a connection mode can not only make the second bone nail prosthesis 2 swing relative to the center line of the hinge joint 14, but also make the second bone nail prosthesis 2 rotate relative to the hinge joint 14, so as to improve the flexibility of relative motion between the metatarsal bone 3 and the phalanx 4. Preferably, the fixed connection mode of the second bone nail 12 with the hinge joint 14 can be threaded connection, so that the fixation of the second bone nail 12 and the hinge joint 14 is stable and easy to install or uninstall. For example, as shown in FIG. 3 and FIG. 4, an end face of the second bone nail 12 in contact with the hinge joint 14 may be provided with a stud 121, and an end face of the hinge joint 14 in contact with the second bone nail 12 may be provided with a threaded hole 142 threadedly connected with the stud 121.

According to the present application, as shown in FIG. 5, the second bone nail prosthesis 2 can further include a needle-shaped portion 22 connected with the spherical concave portion 21 for being implanted into the bone marrow cavity 41 of the phalanx 4. The spherical concave portion 21 further includes an outwardly protruded arc surface 23 connected between the concave spherical surface and the peripheral wall of the needle-shaped portion 22. The arc surface 23 abuts against an end face of the phalanx 4, and the three-dimensional porous structure layer 5 is formed on the arc surface 23. Through this arrangement, in connection with Fig. 2, the needle-shaped portion 22 can be extended into the bone marrow cavity 41 of the phalanx 4 to facilitate its rapid fusion with the bone tissues in the bone marrow cavity 41. Meanwhile, the arc surface 23 outside the phalanx 4 can effectively increase the contact area between its surface and an end face of the phalanx 4, thus effectively ensuring the stability of the fixation of the second nail prosthesis 2 with the phalanx 4. In addition, the height of the second nail prosthesis 2 after matching with the phalanx 4 can be adjusted by adjusting the radian by which the arc surface 23 protrudes outwardly, so as to effectively make up for the length of the bone defect of the phalanx 4. By further forming a three-dimensional porous structure layer 5 on the curved surface 23, the outer surface of the second bone nail prosthesis 2 becomes a porous structure. In this way, the porous structure also endows the arc surface 23 with a better bone ingrowth and bone crawling ability, so that the bone tissues in the bone marrow cavity 41 can quickly grow into its porous structure along the arc surface 23, thus rendering fixation of the second bone nail prosthesis 2 and the phalanx 4 more stable.

In a preferred embodiment shown in FIG. 2, the arc surface 23 of the spherical concave portion may include a first surface 231 extending into the bone marrow cavity 41 of the phalanx 4 and a second surface 232 located outside the bone marrow cavity 41 of the phalanx 4. In particular, the average diameter of the pores of the three-dimensional porous structure layer 5 formed on the first surface 231 is larger than that of the pores in the three-dimensional porous structure layer 5 formed on the second surface 232. Further, the average diameter of the pores in the three-dimensional porous structure layer 5 formed on the first surface 231 ranges from 200 µm to 400 µm for which the porosity in this region can be controlled between 50% and 60%, further preferably 300 µm to 400 µm for which the porosity can be controlled between 50% and 55%. The average diameter of the pores in the three-dimensional porous structure layer 5 formed on the second surface 232 ranges from 100 µm to 200 µm for which the porosity can be controlled between 70% and 80%, and further preferably 100 µm to 150 µm for which the porosity can be controlled between 75% and 80%.

Through the above arrangement, when the diameter of the three-dimensional porous structure layer 5 formed on the first surface 231 ranges from 200 µm to 400 µm, it can better promote bone ingrowth, so as to achieve more stable fixation effect at the initial stage of replacement of the toe joint prosthesis 100. When the diameter of three-dimensional porous structure layer 5 formed on the second surface 232 ranges from 100 µm to 200 µm, the bone crawling growth speed is the fastest, and it can accelerate the growth of bone tissues in the middle and late stages of replacement of the toe joint prosthesis 100, so as to promote a fast recovery for a patient.

In a preferred embodiment, the three-dimensional porous structure layers 5 formed on the first surface 231 and the second surface 232 nay have the same thickness. Preferably, the thickness of the three-dimensional porous structure layer 5 ranges from 1 mm to 2 mm, more preferably 1.5 mm. Through this arrangement, on the one hand, the second bone nail prosthesis 2 has an outer surface which is more conducive to bone ingrowth and bone crawling, and, on the other hand, it endows the second bone nail prosthesis 2 with better stress load carrying, conducting and dispersing capacities, so as to provide the toe joint prosthesis 100 with more stable use effect.

Of course, the three-dimensional porous structure layers 5 formed on the first surface 231 and the second surface 232 may have different thicknesses, which can be specifically designed according to different patients to which the second bone nail prosthesis 1 is applied. For example, when a patient has poor bone ingrowth ability, the thickness of the three-dimensional porous structure layer 5 on the first surface 231 can be configured to be larger than the thickness of the three-dimensional porous structure layer 5 on second surface 232, in order to ensure the strength of the second bone nail prosthesis 2. When a patient has poor bone crawling ability, the thickness of the three-dimensional porous structure layer 5 on the second surface 232 can be configured to be larger than that of the three-dimensional porous structure layer 5 on the first surface 231, in order to ensure the stability of the fixation of the second bone nail prosthesis 1.

In an embodiment shown in FIG. 5, the arc surface 23 may be connected between the concave spherical surface 211 and the peripheral wall in the middle of the needle-shaped portion 22. In this way, the material of the three-dimensional porous structure layer 5 formed on the arc surface 23 can be saved while meeting the requirements of better bone ingrowth and faster bone crawling of the phalanx 4, avoiding wasting of the material. Of course, the arc surface 23 can also be connected between the concave spherical surface 211 and the peripheral wall of the upper middle part of the needle-shaped portion 22, or between the concave spherical surface 211 and the peripheral wall at the lower middle part of the needle-shaped portion 22, which can be set according to the needs of different patients, so as to meet individualized differences of patients.

Preferably, the outer surface of the needle-shaped portion 22 can be configured to be a rough surface. That is, the outer surface of the needle-shaped portion 22 located outside the arc surface 23 can be configure to be a rough surface. The needle-shaped portion 22 can be fixed by bone cement in the bone marrow cavity 41 of the phalanx 4. In this way, the outer surface of the needle-shaped portion 22 has a larger contact area, which is helpful for bone cement to fix the needle-shaped portion 22 with the bone marrow cavity 41 of the phalanx 4 more firmly.

As shown in FIG. 3 and FIG. 5, preferably, the first bone nail 11, the second bone nail 12 and the three-dimensional porous structure layer 5 can be integrally formed, and/or the second bone nail prosthesis 2 and the three-dimensional porous structure layer 5 can be integrally formed. In particular, the three-dimensional porous structure layer 5 can be made of metal powder, which can be titanium alloy, pure titanium or tantalum metal, etc. Preferably, the three-dimensional porous structure layer 5 is made of titanium alloy material, preferably made of Ti6Al4V. Also, all of the first bone nail 11, the second bone nail 12 and the second bone nail prosthesis 2 can be made of titanium alloy. Integrated processing can not only facilitate the processing of the first bone nail prosthesis 1 and the second bone nail prosthesis 2, but also reduce the interaction force between the components of the first bone nail prosthesis 1 and the second bone nail prosthesis 2, so that the first bone nail prosthesis 1 and the second bone nail prosthesis 2 have more stable overall structure and better mechanical strength.

According to the present application, as shown in FIG. 3, a first suture hole 122 extending through the second bone nail 12 is further formed in a side wall of the second bone nail 12, and a second suture hole 143 extending through the hinge joint 14 is formed in one end of the hinge joint 14 close to the second bone nail 12. When a human foot toe joint needs to be repaired due to tumor or comminuted fracture, it is easy to cause serious bone defect of foot toe joints, and serious bone defect is easy to cause joint capsule damage at foot toe joints. Through the above arrangements, soft tissues can be sutured between the first suture hole 122 and the second suture hole 143 to repair or reconstruct the damaged joint capsules, so as to facilitate the postoperative recovery of the patient.

Preferably, a plurality of the first suture hole 122 may be formed on the second bone nail 12, and a plurality of the second suture hole 143 may be formed on the hinge joint 14. Further preferably, as shown in FIG. 3, the number of the first suture holes 122 formed on the second bone nail 12 can be equal to the number of the second suture holes 143 formed on the hinge joint 14, and the first suture holes 122 and the second suture holes 143 are provided close to the outside of the foot toe joint 100. It is preferred that the first suture holes 122 and the second suture holes 143 are aligned in the same vertical line, so that the joint capsule sutured via the first suture hole 122 and the second suture hole 143 more resembles a human joint capsule, further improving the moving ability of the toe joint prosthesis 100 after implantation.

It is further preferred that the diameter of the first suture hole 122 and the second suture hole 143 can range from 2.5 mm to 4 mm. Further, the first suture hole 122 and the second suture hole 143 are specifically through holes bent along the axial direction of the corresponding holes, and the axial length of the through holes with a bending radian can range from 2.5 mm to 40 mm. The specific length value can be determined according to the diameter of a curved suture needle used in the suturing process and the curvature of the curved suture needle, so as to facilitate an operation and better suturing of soft tissues.

Finally, it should be noted that, the above technical solutions are also applicable to the situation where the toe joint prosthesis 100 is applied to a metacarpophalangeal joint replacement for a patient. That is, when the toe joint prosthesis 100 is a metacarpophalangeal joint prosthesis, what is needed is to connect the first bone nail prosthesis 1 of the toe joint prosthesis 100 with a metacarpal bone, and connect the second bone nail prosthesis 2 with a phalanx, so as to replace the metatarsal bone and the phalanx mentioned in the above technical solutions, which is not repeated here.

A fabrication method of a toe joint prosthesis according to the present application includes the following steps:
Step 1, forming a three-dimensional porous structure layer on an outer surface of a first bone nail and/or second bone nail and/or a second bone nail prosthesis;
Step 2, disposing the first bone nail in a bone marrow cavity, disposing the second bone nail outside the bone marrow cavity, and connecting the first bone nail with the second bone nail to form a first bone nail prosthesis; and
Step 3, hinging the first bone nail prosthesis with the second bone nail prosthesis to form a toe joint prosthesis.

Further, the toe joint prosthesis is a foot toe joint prosthesis. In particular, the first bone nail prosthesis is configured to be connected with a metatarsal bone, the second bone nail prosthesis is configured to be connected with a phalanx, the first bone nail is located in the bone marrow cavity of the metatarsal bone, and the second bone nail is located outside the bone marrow cavity of the metatarsal bone and abuts against an end face of the metatarsal bone.

Further, the toe joint prosthesis is a metacarpophalangeal joint prosthesis. In particular, the first bone nail prosthesis is configured to be connected with a metacarpal bone, the second bone nail prosthesis is configured to be connected with a phalangeal bone, the first bone nail is located in the bone marrow cavity of the metacarpal bone, and the second bone nail is located outside the bone marrow cavity of the metacarpal bone and abuts against an end face of the metacarpal bone.

Further, in Step 1, forming a three-dimensional porous structure layer on an outer surface of a first bone nail and/or a second bone nail and/or a second bone nail prosthesis includes the steps of:
scanning the first bone nail and/or the second bone nail and/or the second bone nail prosthesis by using a micro CT, and remodeling the scanned data by using MIMICS to obtain a three-dimensional schematic model;
dividing the three-dimensional schematic model into different regions which are adapted for different growth requirements of the same tissue, and further adjusting the diameter of the pores in individual regions so that different regions have different porosity; and
generating a solid model of the first bone nail and/or the second bone nail and/or the second bone nail prosthesis by using a 3D printing equipment, in which the focus offset parameter of the 3D printing device is adjusted so that a plurality of bumps are formed on the surface of struts in the generated solid model, and the value of the focus offset parameter ranges from 5.8 mA to 6.2 mA.

Further, the average diameter of the pores in the three-dimensional porous structure layer formed on an outer surface of the first bone nail is greater than that of the pores in the three-dimensional porous structure layer formed on an outer surface of the second bone nail.

Further, the first bone nail prosthesis includes a hinge joint fixedly connected with the second bone nail, one end of the hinge joint facing the second bone nail prosthesis is configured as a spherical surface, the second bone nail prosthesis includes a spherical concave portion, and an end face of the spherical concave portion facing the hinge joint is a concave spherical surface matched with the spherical surface.

Further, the second bone nail prosthesis includes a needle-shaped portion connected with the spherical concave portion, and the spherical concave portion further includes an outwardly protruded arc surface connected between the concave spherical surface and the peripheral wall of the needle-shaped portion, and the three-dimensional porous structure layer is formed on the arc surface.

Further, the arc surface of the spherical concave portion includes a first surface configured for extending into the bone marrow cavity and a second surface located outside the bone marrow cavity, in which the average diameter of the pores in the three-dimensional porous structure layer formed on the first surface is greater than the average diameter of the pores in the three-dimensional porous structure layer formed on the second surface.

Further, the average diameter of the pores in the three-dimensional porous structure layer formed on the first surface ranges from 100 µm to 200 µm, and the average diameter of the pores in the three-dimensional porous structure layer formed on the second surface ranges from 200 µm to 400 µm.

Further, a first suture hole extending through the second bone nail is formed on a side wall of the second bone nail, and a second suture hole extending through the hinge joint is formed on one end of the hinge joint near the second bone nail.

Further, the outer surface of the needle-shaped portion is a rough surface.

Further, the first bone nail, the second bone nail and the three-dimensional porous structure layer are integrally formed, and/or, the second bone nail prosthesis and the three-dimensional porous structure layer are integrally formed.

The above embodiments are merely provided for illustrating the technical solutions of the present application, without any limitation thereto. Although the invention has been described in detail with reference to the above-mentioned embodiments, those skilled in the art will understand that, the technical solutions recited in the above-mentioned embodiments can still be modified or some or all of the technical features therein can be equivalently substituted by those skilled in the art. These modifications or substitutions do not make the essence of the corresponding technical solutions depart from the scope of the technical solutions of the embodiments of the present application, which shall be covered in the scope of the claims and the description of the present application. In particular, as long as there is no structural conflict, the technical features mentioned in individual embodiments can be combined in any way. The invention is not limited to the specific embodiments disclosed herein, but includes all technical solutions falling within the scope of the claims.

## Claims

1. A toe joint prosthesis, **characterized in that**, the toe joint prosthesis comprises a first bone nail prosthesis and a second bone nail prosthesis hinged with the first bone nail prosthesis, the first bone nail prosthesis comprises a first bone nail configured to be provided in a bone marrow cavity and a second bone nail connected with the first bone nail and located outside the bone marrow cavity, and the second bone nail is hinged with the second bone nail prosthesis, wherein a three-dimensional porous structure layer is formed on an outer surface of the first bone nail and/or the second bone nail and/or the second bone nail prosthesis.

2. The toe joint prosthesis according to claim 1, **characterized in that**, the toe joint prosthesis is a foot toe joint prosthesis, wherein the first bone nail prosthesis is configured to be connected with a metatarsal bone, the second bone nail prosthesis is configured to be connected with a phalanx, the first bone nail is located in the bone marrow cavity of the metatarsal bone, and the second bone nail is located outside the bone marrow cavity of the metatarsal bone and abuts against an end face of the metatarsal bone.

3. The toe joint prosthesis according to claim 1, **characterized in that**, the toe joint prosthesis is a metacarpophalangeal joint prosthesis, wherein the first bone nail prosthesis is configured to be connected a metacarpal bone, the second bone nail prosthesis is configured to be connected with a phalangeal bone, the first bone nail is located in the bone marrow cavity of the metacarpal bone, and the second bone nail is located outside the bone marrow cavity of the metacarpal bone and abuts against an end face of the metacarpal bone.

4. The toe joint prosthesis according to any one of claims 1-3, **characterized in that**, the three-dimensional porous structure layer comprises a plurality of struts and a plurality of pores formed by staggered connection of the plurality of struts, the pores are communicated with each other and have different average diameters, wherein the average diameter of the pores ranges from 100 µm to 400 µm, and the porosity of the three-dimensional porous structure layer ranges from 50% to 80%.

5. The toe joint prosthesis according to any one of claims 1-3, **characterized in that**, the average diameter of the pores in the three-dimensional porous structure layer formed on the outer surface of the first bone nail is larger than that of the pores in the three-dimensional porous structure layer formed on an outer surface of the second bone nail.

6. The toe joint prosthesis according to any one of claims 1-3, **characterized in that**, the first bone nail prosthesis further comprises a hinge joint fixedly connected with the second bone nail, one end of the hinge joint facing the second bone nail prosthesis is configured as a spherical surface, the second bone nail prosthesis comprises a spherical concave portion, and an end face of the spherical concave portion facing the hinge joint is a concave spherical surface configured to be matched with the spherical surface.

7. The toe joint prosthesis according to claim 6, **characterized in that**, the second bone nail prosthesis further comprises a needle-shaped portion connected with the spherical concave portion, the spherical concave portion further comprises an outwardly protruded arc surface connected between the concave spherical surface and the peripheral wall of the needle-shaped portion, and the three-dimensional porous structure layer is formed on the arc surface.

8. The toe joint prosthesis according to claim 7, **characterized in that**, the arc surface of the spherical concave portion comprises a first surface configured for extending into the bone marrow cavity and a second surface located outside the bone marrow cavity, wherein the average diameter of the pores in the three-dimensional porous structure layer formed on the first surface is greater than the average diameter of the pores in the three-dimensional porous structure layer formed on the second surface.

9. The toe joint prosthesis according to claim 8, **characterized in that**, the average diameter of the pores in the three-dimensional porous structure layer formed on the first surface ranges from 100 µm to 200 µm, and the average diameter of the pores in the three-dimensional porous structure layer formed on the second surface ranges from 200 µm to 400 µm.

10. The toe joint prosthesis according to claim 6, **characterized in that**, a first suture hole extending through the second bone nail is further formed on a side wall of the second bone nail, and a second suture hole extending through the hinge joint is formed on an end of the hinge joint near the second bone nail.

11. The toe joint prosthesis according to any one of claims 7-10, **characterized in that**, an outer surface of the needle-shaped portion is a rough surface.

12. The toe joint prosthesis according to any one of claims 1-3, **characterized in that**, the first bone nail, the second bone nail and the three-dimensional porous structure layer are integrally formed, and/or, the second bone nail prosthesis and the three-dimensional porous structure layer are integrally formed.

13. A fabrication method of a toe joint prosthesis, **characterized in** comprising the following steps:
Step 1, forming a three-dimensional porous structure layer on an outer surface of a first bone nail and/or second bone nail and/or a second bone nail prosthesis;
Step 2, disposing the first bone nail in a bone marrow cavity, disposing the second bone nail outside the bone marrow cavity, and connecting the first bone nail with the second bone nail to form a first bone nail prosthesis; and
Step 3, hinging the first bone nail prosthesis with the second bone nail prosthesis to form a toe joint prosthesis.

14. A fabrication method of a toe joint prosthesis according to claim 13, **characterized in that**, the toe joint prosthesis is a foot toe joint prosthesis, wherein the first bone nail prosthesis is configured to be connected with a metatarsal bone, the second bone nail prosthesis is configured to be connected with a phalanx, the first bone nail is located in the bone marrow cavity of the metatarsal bone, and the second bone nail is located outside the bone marrow cavity of the metatarsal bone and abuts against an end face of the metatarsal bone.

15. A fabrication method of a toe joint prosthesis according to claim 13, **characterized in that**, the toe joint prosthesis is a metacarpophalangeal joint prosthesis, wherein the first bone nail prosthesis is configured to be connected with a metacarpal bone, the second bone nail prosthesis is configured to be connected with a phalangeal bone, the first bone nail is located in the bone marrow cavity of the metacarpal bone, and the second bone nail is located outside the bone marrow cavity of the metacarpal bone and abuts against an end face of the metacarpal bone.

16. A fabrication method of a toe joint prosthesis according to any one of claims 13-15, **characterized in that**, in Step 1, forming a three-dimensional porous structure layer on an outer surface of a first bone nail and/or a second bone nail and/or a second bone nail prosthesis comprises the steps of:
scanning the first bone nail and/or the second bone nail and/or the second bone nail prosthesis by using a micro CT, and remodeling the scanned data by using MIMICS to obtain a three-dimensional schematic model;
dividing the three-dimensional schematic model into different regions which are adapted for different growth requirements of the same tissue, and further adjusting the diameter of the pores in individual regions so that different regions have different porosity; and
generating a solid model of the first bone nail and/or the second bone nail and/or the second bone nail prosthesis by using a 3D printing equipment, wherein the focus offset parameter of the 3D printing device is adjusted so that a plurality of bumps are formed on the surface of struts in the generated solid model, and the value of the focus offset parameter ranges from 5.8 mA to 6.2 mA.

17. A fabrication method of a toe joint prosthesis according to any one of claims 13-15, **characterized in that**, the average diameter of the pores in the three-dimensional porous structure layer formed on an outer surface of the first bone nail is greater than that of the pores in the three-dimensional porous structure layer formed on an outer surface of the second bone nail.

18. A fabrication method of a toe joint prosthesis according to any one of claims 13-15, **characterized in that**, the first bone nail prosthesis further comprises a hinge joint fixedly connected with the second bone nail, one end of the hinge joint facing the second bone nail prosthesis is configured as a spherical surface, the second bone nail prosthesis comprises a spherical concave portion, and an end face of the spherical concave portion facing the hinge joint is a concave spherical surface matched with the spherical surface.

19. A fabrication method of a toe joint prosthesis according to claim 18, **characterized in that**, the second bone nail prosthesis comprises a needle-shaped portion connected with the spherical concave portion, and the spherical concave portion further comprises an outwardly protruded arc surface connected between the concave spherical surface and the peripheral wall of the needle-shaped portion, and the three-dimensional porous structure layer is formed on the arc surface.

20. A fabrication method of a toe joint prosthesis according to claim 19, **characterized in that**, the arc surface of the spherical concave portion comprises a first surface configured for extending into the bone marrow cavity and a second surface located outside the bone marrow cavity, wherein the average diameter of the pores in the three-dimensional porous structure layer formed on the first surface is greater than the average diameter of the pores in the three-dimensional porous structure layer formed on the second surface.

21. A fabrication method of a toe joint prosthesis according to claim 20, **characterized in that**, the average diameter of the pores in the three-dimensional porous structure layer formed on the first surface ranges from 100 µm to 200 µm, and the average diameter of the pores in the three-dimensional porous structure layer formed on the second surface ranges from 200 µm to 400 µm.

22. A fabrication method of a toe joint prosthesis according to claim 21, **characterized in that**, a first suture hole extending through the second bone nail is formed on a side wall of the second bone nail, and a second suture hole extending through the hinge joint is formed on one end of the hinge joint near the second bone nail.

23. A fabrication method of a toe joint prosthesis according to any one of claims 19-21, **characterized in that**, an outer surface of the needle-shaped portion is a rough surface.

24. A fabrication method of a toe joint prosthesis according to any one of claims 13-15, **characterized in that**, the first bone nail, the second bone nail and the three-dimensional porous structure layer are integrally formed, and/or, the second bone nail prosthesis and the three-dimensional porous structure layer are integrally formed.
